**Europäisches Patentamt**

**European Patent Office**

**Office européen des brevets**

(11) Publication number : **0 480 862 A1**

## (12) EUROPEAN PATENT APPLICATION

(21) Application number : **91500109.3**

(22) Date of filing : **30.09.91**

(51) Int. Cl.⁵ : **A61M 5/32, A61B 5/14**

(30) Priority : **09.10.90 ES 9002926**
**31.01.91 ES 9100316**

(43) Date of publication of application :
**15.04.92 Bulletin 92/16**

(84) Designated Contracting States :
**AT BE CH DE DK FR GB GR IT LI LU NL SE**

(71) Applicant : **Ibanez Garcia, Adolfo**
**C/Pedro de Trejo, 3-2 Izd.**
**E-10600 Plasencia (Caceres) (ES)**

(72) Inventor : **Ibanez Garcia, Adolfo**
**C/Pedro de Trejo, 3-2 Izd.**
**E-10600 Plasencia (Caceres) (ES)**

(54) **Single use syringe with automatic safety mechanism.**

(57)    The safety mecanism of the syringe functions as follow :

Remove hood (A), insert the needle (H) into the liquid that is to be taken in, and, exerting slight pressure with the thumb on the outer end of the piston (N), withdraw clip (F) ; if the thumb is then removed from the piston (N) the syringe will automatically be filled up as desired due to the action of spring (G) as it withdraws plunger (L) ; proceed then to eject the liquid by pressing on the piston (N) until male component (D) interacts with female component (K) ; if the thumb is then again removed from the piston (N), shaft (I) together with needle (H) will be withdraw into barrel (M) ; shaft (I) having been withdrawn, the obstructor component (B) will expand. The needle (H) will then remain permanently inside barrel (M), being impossible to move back into position because of obstruction by component (B), and by projection (E) which prevents the piston (N) being withdrawn from the barrel (M).

FIG. 1

EP 0 480 862 A1

# FIG.4

This new invention of a syringe for only one use, with automatic system that propels liquid, occults the needle of the injection and bloks the mechanism is for clinical-sanitary application.

The problem of the present-day syringes is clearly showed in the commercial packig, where the exact words are: use it once and destroy it. the shared use of it created a risk of infection. The same manufacturers of this product are aware of these problems and for this reason they show it like this in the packig of the syringes.

However, the risk is clear when the instructions are not carried out. The means of communicating give us every day an endless number of thrown images of syringes at any public places, probably infected by the drug addicts using.

These syringes do not present ani technique that operates with the man's action, of whom it is not expected to put the lid on the needle and place it in a safe place, as it is many times showed, the problem does not finish at this point, but as it is extremely known, the user of this syringe usually makes use of the public fountains in order to do an urgen cleaning, what is evidently insufficient, and so they give it inmediatly to the other addicts so that they can use it later. Consecuently the number of A.I.D.S' carriers increase.

The present invention, which is the reason for this patent, incorporates an automatic mechanism that operates without the man's action at the stage of absorption of the liquid, occultation of the needle and posterior blocking of this liquid, solving like this all the problems that have been previously described. It is important to consider that the prototype design of this invention has been made according to the smallest model of them all that are commercialized, being liable to be applied to any remaining gamut and size.

The existing syringe only resolves the objetive of its necessity, from a clinical-sanitary point or view. but life show us a whole series of infections that can be contagious at any time by means of a sharp or lesion produced by the needle, due to the user's negligence, or because this syringe is found on the beach with the needle upwards, or even because we are threatened with them to be taken our money away, and also because we can step on then in the parks, when we go gor a walk with our children, or because children pick then up shen they are playing, believing that they have found a missile for their toy soldiers, etc... That is how it is properly explained the raising of the present-day technique, the advantages of the invention and the interest for the industrial-commercial application of this invention into the clinical-sanitary area.

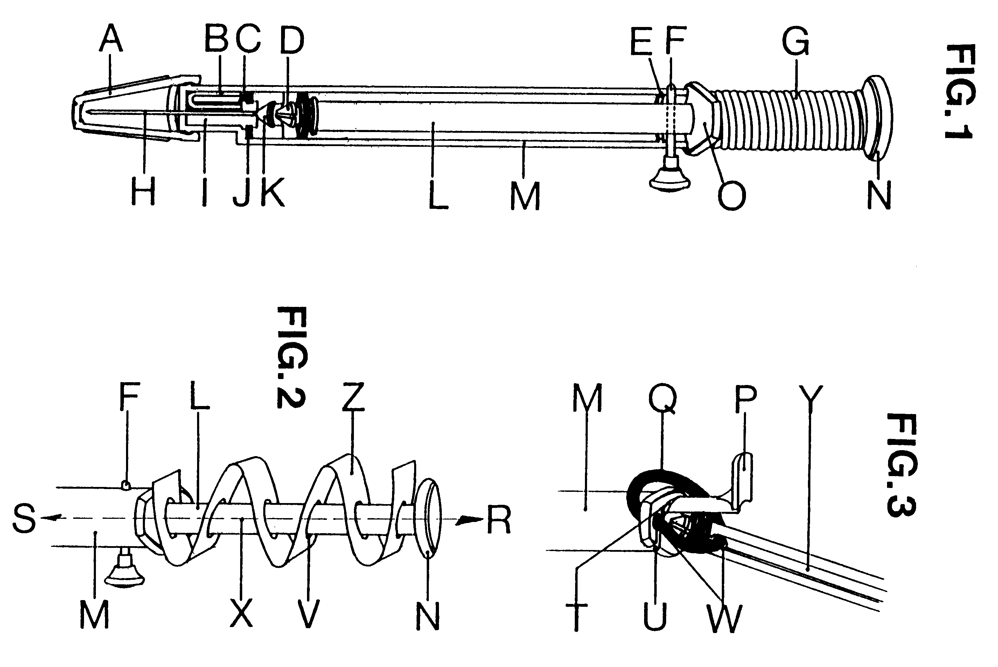

The object invention of this patent, presents the mechanism of a prototype syringe for only one use with three automatic systems: the first one an absortion of the liquid, the second an occultation of the syringe, and finally a blocking of the mechanism, represented in the figure 1.

These three automatic processes are carried out by only one component of the mechanism called motor spring (G) of the figure 1, which is formed by the following components whith the coresponding functions that will be enumerated according to the order of operation of the syringe: Hood of the needle (A) protects this needle until the moment of using it.

Clip of blocking (F), that holds the mechanism with the piston ready to initiate the first stage of absortion of the liquid.

Piston (L), its front part presents a hook of dragging (D) that fits into the cavity of joining of the hook (K) taken place on the base of the needle (I) where is situated the rubber of the needle base (I) because it exerts a pressuere on the walls of the tube of the syringe (M) although it allows the dragging of the mentioned base by the strength of the motor spring (G) towards the inner part of the syringe. The novable sheet of blocking (B) that occupies the space displaced by the base of the needle (I), avoids that the mentioned base occupies once again its initial position and so, it does not allow the needle to go outside.

This mechanism is completed with the leak top of the mechanism (E), made in the inner wall of the tube (M).

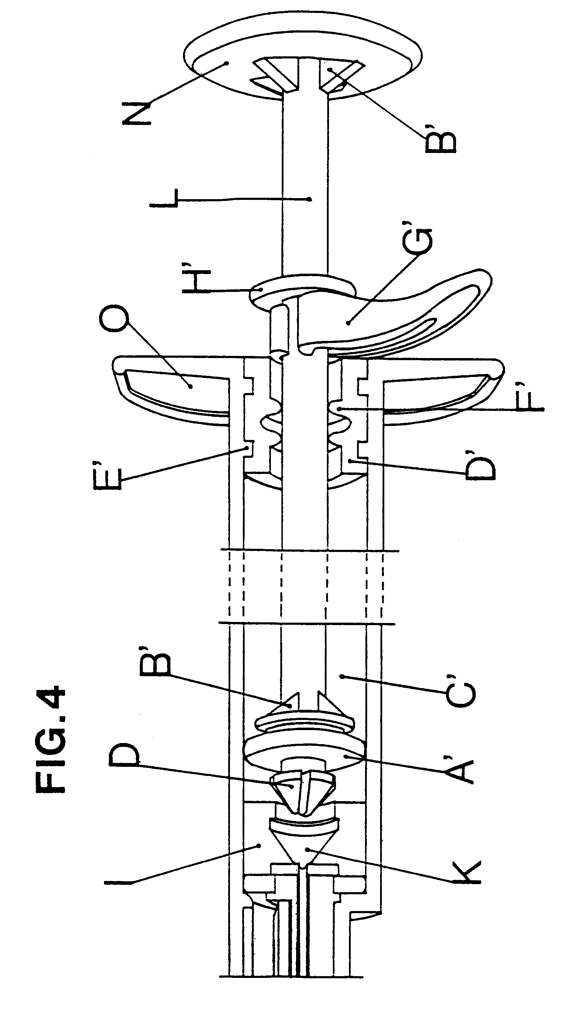

There are many alternatives that enrich this invention represented in the figures 2, 3 and 4.

They present other options of components that performing the same functions as the motor spring (G) of the figure 1, can result more economic for manufacturing and later commercialization of it. The first componente represented in the figure 2, consist of an accordion motor (Z) that can be made of plastic with a suitable thickness or even made of steel with a necessary temper, and it will be situated in the piston (L), in the same way as the mentioned motor spring (G) of the figure 1. Like this, this accordion motor (Z) includes some orifices in its plans, in a vertical way to the sense of movement of the piston (X), represented in the figure 2 by the direction from (R) to (S). The second component, consists of a motor rubber (Q) made in only one part without joinings, that slides along the pipes for the displacement of this motor rubber (W), by a pipe caused in (L) that goes from the dragging hook (D) to the mentioned seating orifice (W), and the other side of the motor rubber will be situated in the seating orifice (W), of the exterior platform of the tube (O), made at both sides of the syringe, by means of some pipes that are the base of the motor rubber (U) of the figure 3.

The third component represented in the figure 4 consist of a vacuum rubber (D') that is situated in the maximum opening of the tube, next to the exterior platform of this one (O). by means of some projections (E') caused inside the capsule. This mechanism includes a top (G') with a thickness that coincides with the measure of approximation of the hook (D) with the cavity (K) in order to avoid the joining of them when the first process of filling of the syringe is carried out.

This top (G') is supported on a circular hoop caused in the piston at the point (H'). It is possible to point out the cylindrical shape of the piston with a smaller thickness than the usual one and it is determined by the orifice of passing made by the hoops (F') of the vacuum rubber (D').

These hoops avoid the inlet of air through this point being able to complete the emptiness, having the rubber of suction (A') as near as possible to the vacuum rubber (D'), and after moving forward with the piston up to the nearest position to the hook (D) with the cavity (K). Finally, this design includes sone supports (B') at both ends of the piston (L) that give a greatest strength to this one at these points.

It is possible to emphasize another component, the top rim of the piston (P), that includes a bevel in the line of detachment of the top rim (T), in order to facilitate its complete separation. This rim has a justified presence because the piston (L) is placed out of the tube of the syringe (M) so that the motor rubber (Q), being placed in a resting position as it is showed in the figure 3, does not waste its qualities that are flexibility and elasticity. Because of what has been said, this mechanism is easy to use being a correct operation as it follows:

Take the hood (A) off, insert the needle (H) into the liquid that is going to be absorbed and holding slightly with the thumb, the exteior platform of the piston (N), take the clip (F) out, straight away automatically and giving up pressing on (N) the syringe will bwe filled up to the desired measure; then we will completely eject the liquid, pressing (N) and so, we will join the hook (D) and the cavity (K), inserting the whole of (I) with the needle (H) into the capsule (M) when giving up operating on the mechanism, being (I) inside the capsule (M), the obstuctor sheet (B) will come out. Thanks to this step, the needle will finally stay inside the capsule (M), being impossible to move back bacause of (B) and the leaving top of the mechanism (E), that obtruct the passage through this point.

The whole of this mechanism, whith the accordion motor spring (Z) of the figure 2, operates exactly in the same way as operating with the motor spring (G) of the figure 1.

A newness in the operation is incorporated with the motor rubber (Q) of the figure 3, in which the clip (F) is replaced by the top rim of the piston (P). In the same way, the mentioned clip (F) is replaced by the top (G'). The rim (P) will be detached when the desired quantity of liquid is inserted, and it serves as the proximity of the hook (D) towards the cavity (K) in the initial process of absorption and it impedes that the joining of (D) and (K) is produced until the rim is detached when filling, and so it can join (D) and (K) on ejecting totally the liquid, being the rest oif the operation as it has been previously descibed.

## Claims

It is claimed as a new and own invention, the exclusive property and commercial use of a:

1) Syringe for only one use, with automatic system that propels liquid, occults the needle of the injection and blocks the mechanism, is characterized by avoiding a second use by hiding the needle of the injection inside the syringe thanks to a special blockade and resulted form theer automatic functions carried out by the motor spring (G) of the figure 1, by the accordion motor (Z) of the figure 2, by the motor rubber (Q) of the figure 3,or well by the vacuum rubber (D') of the figure 4, each one operates separately and independently.

These functions are:A) Absortion of the liquid when the mechanism is released by means of the clip (F) and the strenght of the motor (G),(Z),(Q) or (G'). B) Occultation of the needle of the injection (H), situated on the base (I) which contains the brake rubber (J) and by joining the hook of dragging (D) with the union cavity of this (K), and thanks to the strength of the motor spring, accodion motor, motor rubber, or vacuum rubber. C) Blockade of the mechanism (E), impedes the passage of the needle base (I) thought this point.

Like this, this mechanism is completed with the option that can replace the clip (F) of the figure 1 by the top rim of the piston (P) of the figure 3, in the case of chosing the motor rubber (Q) for the automation of the functiones of the mechanism, or even it can also be replaced by the top (G') of the figure 1 in the case of chosing the mechanism that includes the vacuum rubber (D).

2) A syringe for only one use with automatic system that propels liquids, occults the needle of the injection and blocks the mechanism, according to claims 1, characterized because the mechanism is presented locked thanks to the action of the clip and because the absorption of the liquid is carried out automatically by the motor spring, accordion spring, motor rubber, or vacuum rubber, without the man's action who only controls the speed of the filling.

3) A system according to the claims 1 and 2, characterized by occulting the needle of injection inside the syringe thanks to the strenght of the motor rubber, or vacuum rubber on the base of the needle that carries out the subjection of this one pressing the walls of the capsule, and at the same time, it allows the dragging of this base.

4) A system according to claims 1 to 3, characterized by blocking the mechanism when accupying the moving blocking sheet, the place evacuated by the base of the needle, and so, hindering the return of this one to the starting point, and it is also characterized by incorporatig the syringe near the accomodation of the clip, a leaving top of the mechanism that consist of a circular projection like a ring with a central orifice, smaller than the radius of the base of the needle.

5) A system according to claims 1 to 4, characterized by offering the option showed in the figure 3, that allows the motor rubber to be stopped, without losing the qualities of flexibility an elasticity during its permanence in the container, and it also incorporates the top rim of the piston that replaces the functions of the clip in the nearest measure to the hook towards the union cavity of this one, being detached wuen the syringre is filled by the desired liquid.

6) A system sccording to claims 1 to 5, chareacterized by offering a vaccum rubber (D') with circular hoop (F') that impede the inlet of the air though this point, being able to produce a vacuum behind the rubber of suction (A') when this one is as nearest as possible from (D') and even, when it moves toward with tue piston (L) pressing (N) until the hook (D) comes to the cavity (K). The joining of (D) and (K) will not be carried out until the top (G') is released from the piston (L). This system is characterized by presenting a circular hoop (H') made in (L), and due to the fact that (L) in every sense is cylindrical and has got a thickness that is slightly superior to the circle formed by the hoops (F'), this piston includes some supports at both ends of this one, on the point (B') that give it a greater strength.

7) A system accordinf to claims 1 to 6, characterized by including a system based on pipes drawn in the figure 3, that allows the mounting of the motor rubber in the piston in only one piece without joinings, through this pipe that goes from the hook to the seating of the rubber in the piston and at the same time, in the exterior platform of the tube of the syringe through the pipes produced at both sides and that allows the internment of this motor rubber until the seating orifices of the rubber in this platform.

8) A syringe for only one use with automatic system that propels liquids, occults the needle of the injection and blocks the mechanism, characterized by presenting three options of components that carry out the automatic function of this syringe correctly described in the main part of this report, and claims with three drawings, all of then included in eight pages, that mean the presentation of a patent number 9100316 in the patent Office of the Industrial Property Register in Madrid, with the priority date on october 19th 1.990.

9) A system according to claims 1 to 8 characterized by presenting four independent options of operation, which, doing the same work with the mechanism and in the same way, are those described in the main part of this report and in the drawings 1,2,3 and 4.

10) A Syringe for only one use, with automatic system that propels liquids, occults the needle of the injection and blocks the mechanism, as it is descibed in the main part of this report, and it includes the description, claims and a summary that contain of eleven pages, written on only one side, and with four drawings, three of which are designed in page number ten and the fourth one in page number eleven.

# FIG. 1

# FIG. 3

# FIG. 2

FIG.4

**European Patent Office**

## PARTIAL EUROPEAN SEARCH REPORT

which under Rule 45 of the European Patent Convention shall be considered, for the purposes of subsequent proceedings, as the European search report

Application Number

EP 91 50 0109

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 347 742 (VENTURINI)<br>* Abstract; figures 1-3,46,47 * | 1-3 | A 61 M 5/32<br>A 61 B 5/14 |
| Y | | 4 | |
| Y | EP-A-0 276 160 (E.R. SQUIBB & SONS, INC.)<br>* Abstract; figures 3,4 * | 4 | |
| A | US-A-4 698 055 (SEALFON)<br>* Abstract; figures 2,4 * | 1 | |

TECHNICAL FIELDS SEARCHED (Int. Cl.5)

A 61 M
A 61 B

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 1-7
Claims searched incompletely :
Claims not searched : 8-10
Reason for the limitation of the search:

See Rule 29.6

| Place of search | Date of completion of the search | Examiner |
|---|---|---|
| THE HAGUE | 10-01-1992 | ZEINSTRA H.S.J.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)